# EUROPÄISCHE PATENTSCHRIFT

(11) **EP 3 837 983 B1**
(45) Veröffentlichungstag und Bekanntmachung des Hinweises auf die Patenterteilung: **30.04.2025**
(21) Anmeldenummer: 19216768.2
(22) Anmeldetag: 17.12.2019
(51) Int. Cl.: A23C 1/12, A23C 9/142, A23C 21/00, A23L 33/00, A61Q 19/00, A61K 8/64

(54) **VERFAHREN ZUR HERSTELLUNG EINES DEMINERALISIERTEN SÜSSMOLKEPULVERS**
METHOD FOR THE PRODUCTION OF DEMINERALIZED SWEET WHEY POWDER
PROCÉDÉ DE PRODUCTION D'UNE POUDRE DE LACTOSÉRUM DOUX DÉMINÉRALISÉ

(43) Veröffentlichungstag der Anmeldung: 23.06.2021
(73) Patentinhaber: DMK Deutsches Milchkontor GmbH, 27404 Zeven (DE)
(72) Erfinder: DÖRING, Sven-Rainer, 27404 Zeven (DE)
(74) Vertreter: Fabry, Bernd

(56) Entgegenhaltungen:
- EP-A1- 2 839 749
- WO-A1-2004/057973
- WO-A1-2006/135983
- WO-A1-96/23417
- WO-A1-96/32021
- US-A1- 2009 142 459
- GERMANO MUCCHETTI ET AL: "The pre-concentration of milk by nanofiltration in the production of Quarg-type fresh cheeses", LAIT, 1 January 2000 (2000-01-01), pages 43 - 50, XP055087166, Retrieved from the Internet <URL:http://lait.dairy-journal.org/articles/lait/pdf/2000/01/L0107.pdf> [retrieved on 20131107], DOI: 10.1051/lait:2000106

## Beschreibung

### GEBIET DER ERFINDUNG

Die Erfindung befindet sich auf dem Gebiet der Milchwirtschaft und betrifft ein Verfahren zur Herstellung eines demineralisiertes Süßmolkepulver mit hohem Proteinanteil.

### TECHNOLOGISCHER HINTERGRUND

Molke ist die wässrige grünlich-gelbe Restflüssigkeit, die bei der Käseherstellung entsteht. Sie ist der flüssige Teil, der nach der Gerinnung der Milch zu Käse oder Quark abgesondert werden kann. Es gibt zwei Sorten von Molke: die *Süßmolke* (auch *Labmolke*), die entsteht, wenn man Milch mit Lab zur Käseherstellung dicklegt, und die *Sauermolke,* die entsteht, wenn Milch durch Milchsäurebakterien fermentiert wird.

Molke besteht zu 94 % aus Wasser, zu 4 bis 5 % aus Milchzucker und ist nahezu fettfrei. Außerdem enthält sie Milchsäure, die Vitamine B₁, B₂ (dies bewirkt die grünliche Farbe) und B₆ sowie Kalium, Calcium, Phosphor und andere Mineralstoffe, doch vor allem 0,4 bis 1 % Molkenprotein. Molke enthält deutlich weniger Eiweiß als Milch. Insbesondere enthält sie anders als Milch kein Kasein. In Milch ist das Kasein hingegen das Haupteiweiß. Süßmolke stellt daher eine wertvolle Proteinquelle dar. Entsprechende Produkte gelangen als pulverförmige Konzentrate in den Handel.

Zur Herstellung entsprechender Süßmolkepulver wird Süßmolke üblicherweise einem dreistufigen Prozess unterworfen. Dieser besteht aus einer Nanofiltration gefolgt von einer Elektrodialyse und einem Ionenaustauscher. Nachteilig ist, dass die Elektrodialyse bei einer Temperatur im Bereich von 25 bis 40 °C durchgeführt werden muss, da dies zum Wachstum von Keimen führt. Der Ionenaustauscher hat die Aufgabe, bivalente Ionen, die die Elektrodialyse überstanden haben, abzutrennen ("polishing"). Auch dieser Schritt ist von Nachteil, weil der Ionenaustauscher über eine große Fläche verfügt, die schwierig zu reinigen ist und ebenfalls Raum für starkes Keimwachstum bietet. Auf diese Weise kann es daher leicht zu einer Rekontaminierung des Produktes kommen.

### RELEVANTER STAND DER TECHNIK

Gegenstand der US 2009/142459 A1 ist ein Verfahren zur Herstellung eines demineralisierten Molkepulvers, welches die folgenden Schritte umfasst: (a) Aufkonzentrieren der Molke; (b) Demineralisierung mit Hilfe eines Kationenaustauschers; (c) Elektrodialyse; (d) lonenaustauscherbeh andlung ("Polisher"), und (e) Sprühtrocknung.

Aus der WO 2004 057973 A1 ist ein Verfahren zur Herstellung von Molkenproteinkonzentraten bekannt, bei dem man Süßmolke filtriert, pasteurisiert, kristallisiert und schließlich durch Sprühtrocknung entwässert.

Gegenstand der WO 2006 135983 A1 ist ein Verfahren zur Gewinnung eines Kalium-reichen Mineralstoffgemischs, bei dem Molke durch Filtration demineralisiert wird und das Retentat anschließend über einen Ionenaustauscher vom Calciumphosphat befreit wird.

### AUFGBE DER ERFINDUNG

Die Aufgabe der vorliegenden Erfindung hat daher darin bestanden, ein alternatives Verfahren zur Herstellung von demineralisiertem Süßmolkepulver zur Verfügung zu stellen, das frei von den geschilderten Nachteilen ist und insbesondere zu Produkten mit einstellbarem, vorzugsweise höherem Proteinanteil führt. Das Verfahren sollte zudem zu keimarmen bzw. keimfreien Produkten mit hoher Geschmacksqualität führen.

### BESCHREIBUNG DER ERFINDUNG

Gegenstand der Erfindung ist ein Verfahren zur Herstellung eines demineralisierten Süßmolkepulvers, umfassend oder bestehend aus den folgenden Schritten:
(a) Bereitstellen von Molke,
(b) Dia- Ultrafiltration der Molke unter Erhalt eines erstes proteinreiches Retentats R1 sowie eines ersten Permeats P1;
(c) Nanofiltration des Permeat P1 aus Schritt (b) unter Erhalt eines Lactose und Mineralien enthaltenden zweiten Retentats R2 sowie eines zweiten Permeats P2, wobei die Nanofiltration mit einer Membran durchführt, die einen mittleren Porendurchmesser von etwa 100 bis etwa 2.000 Dalton aufweist;
(d) Temperaturbehandlung des Retentats R2 aus Schritt (c);
(e) Filtration des temperaturbehandelten Produktes aus Schritt (d) unter Erhalt eines dritten phosphathaltigen Retentats R3 sowie eines dritten Permeats P3;
(f) Vermischen des dritten Permeats P3 aus Schritt (e) mit dem proteinreichen Retentat R1 aus Schritt (b); und
(g) Entwässern der Mischung aus Schritt (f).

Überraschenderweise wurde gefunden, dass durch die spezielle Abfolge der Verfahrensschritte ein demineralisiertes Süßmolkepulver mit einstellbarem Proteingehalt ohne die Gefahr einer Verkeimung erhalten wird. Dadurch, dass mal mehr und mal weniger Retentat R2 zurückgeführt wird, können angepasste Proteingehalte eingestellt werden. Das Produkt weist einen Mineraliengehalt von weniger als 2 Gew.-% und insbesondere weniger als 1 Gew.-% auf und erfüllt daher auch die geschmacklichen Vorgaben.

### FILTRATIONSVERFAHREN

Das erfindungsgemäße Verfahren sieht verschiedene Filtrationsschritte vor, nämlich eine Dia- oder Ultrafiltration in Schritt (b), eine Nanofiltration in Schritt (c) sowie eine weitere Ultrafiltration oder Mikrofiltration in Schritt (e). Im ersten Filtrationsschritt geht es darum, große und besonders temperaturlabile Proteine als Retentat aus der Molke abzutrennen und diese Fraktion erst später wieder zuzusetzen. Außerdem können bei der Diafiltration auch Salze ausgewaschen werden. Hierfür kann das Permeat der nachfolgenden Nanofiltration oder Wasser verwendet werden. Bei der sich anschließenden Nanofiltration des Permeats werden über das Permeat Mineralien - im Wesentlichen Natrium und Kalium - abgetrennt, die ansonsten dem Pulver einen unerwünschten metallischen Beigeschmack verleihen würden. Außerdem wird die Lactose entfernt; der entsprechende Seitenstrom kann anschließend separat aufgearbeitet werden. Mittels einer zweiten Ultra- oder Mikrofiltration wird nach der Temperaturbehandlung das Calciumphosphat abgetrennt.

Ultra- und Nanofiltration sind Filtrationsverfahren aus dem Bereich der Membrantechnik, mit der sich makromolekulare Substanzen und kleine Partikel aus einem Medium abtrennen und aufkonzentrieren lassen. Man unterscheidet Mikrofiltration, Ultrafiltration und Nanofiltration über den Grad der Abtrennung. Liegt die Ausschlussgrenze (oder auch *"Cut-off"*) bei 100 nm oder darüber, spricht man von Mikrofiltration. Liegt die Ausschlussgrenze in dem Bereich zwischen 2-100 nm, bezeichnet man dies als Ultrafiltration. Bei der Nanofiltration liegt die Ausschlussgrenze unterhalb von 2 nm. In jedem dieser Fälle handelt es sich um rein physikalische, d.h. mechanische Membrantrennverfahren, die nach Prinzip des mechanischen Größenausschlusses arbeiten: alle Partikel in den Fluiden, die größer als die Membranporen sind, werden von der Membran zurückgehalten. Treibende Kraft in beiden Trennverfahren ist der Differenzdruck zwischen Zulauf und Ablauf der Filterfläche, der bei Ultra- und Mikrofiltration zwischen 0,1 und 10 bar und bei der Nanofiltration bis ca. 40 bar liegt.

Die Ausschlussgrenzen von Ultrafiltrationsmembranen werden auch in Form des *NMWC* (englisch: Nominal Molecular Weight Cut-Off, auch *MWCO,* Molecular Weight Cut Off, Einheit: Dalton) angegeben. Er ist definiert als die minimale Molekülmasse globulärer Moleküle, welche durch die Membran zu 90% zurückgehalten werden. In der Praxis sollte der NMWC mindestens 20 % niedriger sein als die Molmasse des abzutrennenden Moleküls. Weitere qualitative Aussagen über die Filtration lassen sich anhand des *Flux* (Wasserwert) (Transmembranfluss oder Durchtrittsrate) machen. Dieser verhält sich im Idealfall proportional zum Transmembrandruck und reziprok zum Membranwiderstand. Diese Größen werden sowohl von den Eigenschaften der verwendeten Membran als auch durch Konzentrationspolarisation und eventuell auftretendes Fouling bestimmt. Die Durchtrittsrate wird auf 1 m² Membranfläche bezogen. Ihre Einheit ist l/(m²h bar).

Für die Ultrafiltration haben sich Membranen als besonders geeignet erwiesen, die einen Porendurchmesser im Bereich von etwa 1.000 bis etwa 50.000 und vorzugsweise etwa 5.000 bis etwa 25.000 Dalton besitzen. Die Nanofiltration bevorzugt Porendurchmesser im Bereich von etwa 150 bis 1.000 Dalton.

Der Werkstoff der Filterfläche - sowohl bei der Ultra- als auch der Nanofiltration - kann Edelstahl, Polymerwerkstoffen, Keramik, Aluminiumoxid oder textilen Gewebe darstellen. Es gibt verschiedene Erscheinungsformen der Filterelemente: Kerzenfilter, Flachmembranen, Spiralwickelmembranen, Taschenfilter und Hohlfasermodule, die im Sinne der vorliegenden Erfindung alle grundsätzlich geeignet sind. Vorzugsweise werden jedoch Spiralwickelmembranen aus Polymerwerkstoffen oder Kerzenfilter aus Keramik oder Aluminiumoxid eingesetzt, wobei sich die erste Ausführungsform für Ultra- und Nanofiltration und die zweite für die Mikrofiltration besonders bevorzugt erwiesen hat.

Auch die Mikro- oder Diafiltration gehört zu den Membrantrennverfahren. Der wesentliche Unterschied zu Ultra- und Nanofiltration besteht in den größeren Porendurchmessern von mehr als 0,1 µm, speziell von etwa 0,1 bis 1,4 µm entsprechend etwa 10.000 bis etwa 800.000 Dalton.

Die Angaben zum Cut-off überschneiden sich im Grenzbereich, so dass sich beispielsweise eine Membran mit einem Porendurchmesser von etwa 2.000 Dalton sowohl für eine Ultra- als auch eine Nanofiltration eignen kann.

Alle genannten Filtrationsverfahren können im Sinne der vorliegenden Erfindung unabhängig voneinander "heiß" oder "kalt", d.h. im Temperaturbereich von etwa 10 bis etwa 60 °C durchgeführt werden. Bevorzugt ist es jedoch, bei Temperaturen im niedrigen Bereich von etwa 10 bis etwa 20 °C zu arbeiten.

### TEMPERATURBEHANDLUNG

Im Anschluss an die Nanofiltration wird das erhaltene Retentat einer Temperaturbehandlung unterzogen, um das Produktkeimfrei zu machen. Als keimfrei wird ein Produkt bezeichnet, welches pro Milliliter weniger als 1.000 (thermophile und mesophile) Keime aufweist. Bei der Temperaturbehandlung handelt es sich um eine Ultrahocherhitzung, die typischerweise bei etwa 70 bis etwa 90 °C über einen Zeitraum von etwa 10 bis etwa 30 Minuten und insbesondere etwa 15 bis etwa 20 Minuten durchgeführt wird. Diese Maßnahme führt u.a. dazu, dass Salze ausfallen und wird vorzugsweise in einem Rührkessel durchgeführt.

### TROCKNUNG

Das temperaturbehandelte Produkt wird wie beschrieben einer Mikro- bzw. Ultrafiltration unterworfen und dabei vorhandenes Magnesiumphosphat entfernt. Alternativ ist es auch möglich, das Phosphat zu fällen und dann abzufiltrieren. Das phosphatfreie Produkt wird wie beschrieben mit dem Retentat aus Schritt (a), welches die großen und besonders temperaturempfindlichen Proteine enthielt, vereinigt. Anschließend erfolgt die Trocknung der Mischung.

Vorzugsweise kommt dabei die Sprühtrocknung zum Zuge, wobei die Temperatur im Einlass typischerweise etwa 180 bis etwa 260 °C und am Ausgang etwa 80 bis etwa 105 °C beträgt. Die Fraktion bedarf daher keiner Kühlung bevor sie in den Sprühturm gelangt. Temperaturen von 60 bis 70 °C sind dabei sogar bevorzugt, da auf diese Weise die Gefahr verringert wird, dass Teil der Proteine denaturieren. Alternativ können die Produkte auch durch Gefriertrocknung entwässert werden. Der Restwassergehalt beträgt dabei maximal 5 Gew.-% und vorzugsweise etwa 2 bis etwa 3 Gew.-%.

Dem Produkt können vor, vorzugsweise aber nach dem Versprühen auch weitere Zusatzstoffe zugesetzt werden, wie beispielsweise Lactoferrin, Lecithine, Vitamine oder Lebensmittelemulgatoren [EP 1314367 A1**,** NESTLE] und dergleichen. Man erhält schließlich ein demineralisiertes Süßmolkenpulver, das einen Proteinanteil von etwa 50 bis etwa 95 Gew.-%, vorzugsweise von etwa 85 bis etwa 92 Gew.-% aufweist.

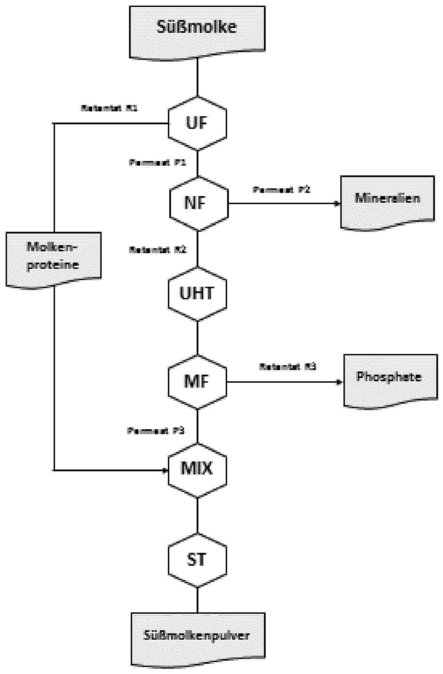

Das erfindungsgemäße Verfahren wird auch im Fließbild nach **Abbildung 1** näher erläutert. Dabei bedeuten UF = Ultrafiltration, NF = nanofiltration, UHT = Ultrahocherhitzung, MF = Mikrofiltration, MX = Vermischung und ST = Sprühtrocknung.

### GEWERBLICHE ANWENDBARKEIT

Ein weiterer Gegenstand der Erfindung betrifft die Verwendung des erfindungsgemäßen demineralisierten Süßmolkepulvers oder des entsprechenden Verfahrensproduktes als Nahrungsmittel, speziell als Proteinquelle für die Kleinkindernährung sowie zur Herstellung von kosmetischen Produkten, speziell Cremes und Lotionen für die Pflege von Haut und Haaren, in die sich die Pulver problemlos einarbeiten lassen.

### BEISPIELE

### BEISPIEL 1

### Herstellung eines demineralisierten Süßmolkepulvers gemäß Erfindung

Im Technikumsbetrieb wurden 1.000 Liter Süßmolke bei 10 °C über eine Ultrafiltrationseinheit geleitet, die mit einer Keramikmembran mit einem mittleren Porendurchmesser von 25.000 Dalton ausgestattet war. Es wurde ein proteinreiches Retentat R1 und ein verarmtes Permeat P1 erhalten. Das Permeat P1 wurde bei gleichbleibender Temperatur auf eine Nanofiltrationsanlage mit einer Spiralwickelmembran mit einem mittleren Porendurchmesser von 700 Dalton gegeben. Es wurde ein Lactose und Mineralien enthaltendes Retentat R2 erhalten sowie ein Permeat P2. Das Retentat R2 wurde für etwa 20 Minuten auf 78 °C und dann bei 55 °C auf eine Mikrofiltrationseinheit gegeben, die mit einer Keramikmembran mit einem mittleren Porendurchmesser von 0,2 µm versehen war. Bei etwa 15 °C wurde ein phosphathaltiges drittes Retentat R3 und ein drittes Permeat P3 erhalten. Die Fraktionen P3 und R1 wurden in einer Mischeinheit vereinigt, über einen Wärmetauscher auf etwa 80 °C vorgewärmt und dann bei 180°C Kopftemperatur über einen Turm versprüht. Das resultierende Pulver wies einen Proteingehalt von 13,7 Gew.-%, einen Aschegehalt von 1,2 Gew.-% und eine Restfeuchte von etwa 3 Gew.-% auf.

### VERGLEICHSBEISPIEL V1 Herstellung eines demineralisierten Süssmolkepulvers nach dem Stand der Technik

Analog Beispiel 1 wurden 1.000 Liter Süßmolke bei 12 °C auf eine Nanofiltrationsanlage mit einer Spiralwickelmembran mit einem mittleren Porendurchmesser von 2.000 Dalton gegeben. Das Lactose und Mineralien enthaltende Retentat wurde auf 35 °C angewärmt und einer Elektrodialyse unterworfen. Anschließend wurde es auf 15 °C abgekühlt über einen Ionenaustauscher geführt und wie in Beispiel 1 versprüht. Das resultierende Pulver wies einen deutlich geringeren Proteingehalt von 11,5 Gew.-%, einen Aschegehalt von 1,5 Gew.-% und eine Restfeuchte von etwa 3 Gew.-% auf.

## Patentansprüche

1. Verfahren zur Herstellung eines demineralisierten Süßmolkepulvers, umfassend oder bestehend aus den folgenden Schritten:
(a) Bereitstellen von Molke,
(b) Dia- oder Ultrafiltration der Molke unter Erhalt eines erstes proteinreiches Retentats R1 sowie eines ersten Permeats P1;
(c) Nanofiltration des Permeat P1 aus Schritt (b) unter Erhalt eines Lactose und Mineralien enthaltenden zweiten Retentats R2 sowie eines zweiten Permeats P2, wobei die Nanofiltration mit einer Membran durchführt, die einen mittleren Porendurchmesser von etwa 100 bis etwa 2.000 Dalton aufweist;
(d) Temperaturbehandlung des Retentats R2 aus Schritt (c);
(e) Filtration des temperaturbehandelten Produktes aus Schritt (d) unter Erhalt eines dritten phosphathaltigen Retentats R3 sowie eines dritten Permeats P3;
(f) Vermischen des dritten Permeats P3 aus Schritt (e) mit dem proteinreichen Retentat R1 aus Schritt (b); und
(g) Entwässern der Mischung aus Schritt (f).

2. Verfahren nach Anspruch 1, **dadurch gekennzeichnet, dass** man die Ultrafiltration gemäß Schritt (b) mit einer Membran durchführt, die einen mittleren Porendurchmesser von etwa 1.000 bis etwa 50.000 Dalton aufweist.

3. Verfahren nach mindestens einem der Ansprüche 1 bis 2, **dadurch gekennzeichnet, dass** man die Ultrafiltration gemäß Schritt (b) bzw. die Nanofiltration gemäß Schritt (c jeweils unabhängig voneinander bei Temperaturen im Bereich von etwa 10 bis etwa 60 °C durchführt.

4. Verfahren nach mindestens einem der Ansprüche 1 bis 3, **dadurch gekennzeichnet, dass** man die Temperaturbehandlung gemäß Schritt (d) bei einer Temperatur im Bereich von etwa 70 bis etwa 90 °C durchführt.

5. Verfahren nach mindestens einem der Ansprüche 1 bis 4, **dadurch gekennzeichnet, dass** man die Temperaturbehandlung gemäß Schritt (d) über einen Zeitraum von etwa 10 bis etwa 30 Minuten durchführt.

6. Verfahren nach mindestens einem der Ansprüche 1 bis 5, **dadurch gekennzeichnet, dass** man das temperaturbehandelte Produkt aus Schritt (d) einer Ultrafiltration oder einer Mikrofiltration unterwirft.

7. Verfahren nach Anspruch 6, **dadurch gekennzeichnet, dass** man die Ultra- bzw. Mikrofiltration gemäß Schritt (e) mit einer Membran durchführt, die einen mittleren Porendurchmesser von etwa 10.000 bis etwa 800.000 Dalton aufweist.

8. Verfahren nach den Ansprüchen 6 und/oder 7, **dadurch gekennzeichnet, dass** man die Ultra- bzw. Mikrofiltration gemäß Schritt (e) bei einer Temperatur im Bereich von etwa 10 bis etwa 60 °C durchführt.

9. Verfahren nach mindestens einem der Ansprüche 1 bis 8, **dadurch gekennzeichnet, dass** man das Mischprodukt aus Schritt (f) einer Gefriertrocknung oder einer Sprühtrockung unterwirft.

10. Verfahren nach mindestens einem der Ansprüche 1 bis 9, **dadurch gekennzeichnet, dass** man die Sprühtrocknung gemäß Schritt (g) bei Temperaturen von 180 bis etwa 260 °C - gemessen bei der Aufgabe auf den Sprühturm - durchführt.

11. Verfahren nach mindestens einem der Ansprüche 1 bis 10, **dadurch gekennzeichnet, dass** man ein demineralisiertes Süßmolkenpulver herstellt, das einen Proteinanteil von etwa 11 bis etwa 18 Gew.-% und einen Mineralienanteil von weniger als 2 Gew.-% aufweist.

## Claims

1. Process for producing a demineralized sweet whey powder, comprising or consisting of the following steps:
(a) providing whey,
(b) diafiltration or ultrafiltration of the whey to obtain a first protein-rich retentate R1 and a first permeate P1,
(c) nanofiltration of the permeate P1 from step (b) to obtain a second retentate R2 containing lactose and minerals and a second permeate P2, wherein the nanofiltration is performed using a membrane with an average pore diameter of approximately 100 to 2,000 Daltons,
(d) temperature treatment of the retentate R2 from step (c),
(e) filtration of the temperature -treated product from step (d) to obtain a third phosphate-containing retentate R3 and a third permeate P3,
(f) mixing the third permeate P3 from step (e) with the protein-rich retentate R1 from step (b), and
(g) dehydrating the mixture from step (f).

2. Process according to claim 1, **characterized in that** the ultrafiltration according to step (b) is carried out with a membrane having an average pore diameter of about 1,000 to 50,000 Daltons.

3. Process according to at least one of claims 1 to 2, **characterised in that** the ultrafiltration according to step (b) and the nanofiltration according to step (c) are each carried out independently of one another at temperatures in the range from about 10 to about 60°C.

4. Process according to at least one of claims 1 to 3, **characterised in that** the temperature treatment according to step (d) is carried out at a temperature in the range from about 70 to about 90°C.

5. Method according to at least one of claims 1 to 4, **characterised in that** the temperature treatment according to step (d) is carried out over a period of about 10 to about 30 minutes.

6. Process according to at least one of claims 1 to 5, **characterised in that** the temperature-treated product from step (d) is subjected to ultrafiltration or microfiltration.

7. Process according to claim 6, **characterised in that** the ultrafiltration or microfiltration according to step (e) is carried out with a membrane which has an average pore diameter of about 10,000 to about 800,000 Daltons.

8. Process according to claims 6 and/or 7, **characterised in that** the ultrafiltration or microfiltration according to step (e) is carried out at a temperature in the range from about 10 to about 60°C.

9. Process according to at least one of claims 1 to 8, **characterised in that** the mixed product from step (f) is subjected to freeze-drying or spray-drying.

10. Process according to at least one of claims 1 to 9, **characterised in that** the spray drying according to step (g) is carried out at temperatures of 180 to about 260°C - measured at the feed onto the spray tower.

11. Process according to at least one of claims 1 to 10, **characterised in that** a demineralised sweet whey powder is produced which has a protein content of about 11 to about 18% by weight and a mineral content of less than 2% by weight.

## Revendications

1. Procédé de préparation d'une poudre de lactosérum doux déminéralisé, comprenant ou consistant en les étapes suivantes :
(a) mise à disposition de lactosérum,
(b) diafiltration ou ultrafiltration du lactosérum pour obtenir un premier rétentat R1 riche en protéines ainsi qu'un premier perméat P1 ;
(c) nanofiltration du perméat P1 de l'étape (b) pour obtenir un deuxième rétentat R2 contenant du lactose et des minéraux, ainsi qu'un deuxième perméat P2, la nanofiltration étant effectuée avec une membrane ayant un diamètre moyen de pores d'environ 100 à environ 2000 daltons ;
(d) traitement thermique du rétentat R2 de l'étape (c) ;
(e) filtration du produit traité par la température de l'étape (d) pour obtenir un troisième rétentat R3 contenant du phosphate ainsi qu'un troisième perméat P3 ;
(f) mélange du troisième perméat P3 de l'étape (e) avec le rétentat R1 riche en protéines de l'étape (b) ; et
(g) déshydrater le mélange de l'étape (f).

2. Procédé selon la revendication 1, **caractérisé en ce que** l'on réalise l'ultrafiltration selon l'étape (b) avec une membrane dont le diamètre moyen des pores est compris entre environ 1000 et environ 50 000 daltons.

3. Procédé selon au moins l'une des revendications 1 à 2, **caractérisé en ce que** l'on réalise l'ultrafiltration selon l'étape (b) ou la nanofiltration selon l'étape (c), respectivement, indépendamment les unes des autres, à des températures comprises entre environ 10 et environ 60°C.

4. Procédé selon au moins l'une des revendications 1 à 3, **caractérisé en ce que** l'on réalise le traitement thermique selon l'étape (d) à une température comprise entre environ 70 et environ 90°C.

5. Procédé selon au moins l'une des revendications 1 à 4, **caractérisé en ce que** l'on effectue le traitement thermique selon l'étape (d) pendant une durée comprise entre environ 10 et environ 30 minutes.

6. Procédé selon au moins l'une des revendications 1 à 5, **caractérisé en ce que** l'on soumet le produit traité thermiquement de l'étape (d) à une ultrafiltration ou à une microfiltration.

7. Procédé selon la revendication 6, **caractérisé en ce que** l'on réalise l'ultrafiltration ou la microfiltration selon l'étape (e) avec une membrane dont le diamètre moyen des pores est compris entre environ 10 000 et environ 800 000 daltons.

8. Procédé selon les revendications 6 et/ou 7, **caractérisé en ce que** l'on réalise l'ultrafiltration ou la microfiltration selon l'étape (e) à une température comprise entre environ 10 et environ 60°C.

9. Procédé selon au moins l'une des revendications 1 à 8, **caractérisé en ce que** l'on soumet le produit mixte de l'étape (f) à une lyophilisation ou à un séchage par atomisation.

10. Procédé selon au moins l'une des revendications 1 à 9, **caractérisé en ce que** l'on effectue le séchage par atomisation selon l'étape (g) à des températures de 180 à environ 260 °C - mesurées lors de l'application sur la tour d'atomisation.

11. Procédé selon au moins l'une des revendications 1 à 10, **caractérisé en ce que** l'on prépare une poudre de lactosérum doux déminéralisé présentant une teneur en protéines d'environ 11 à environ 18 % en poids et une teneur en minéraux inférieure à 2 % en poids.
